(19) **Europäisches Patentamt / European Patent Office / Office européen des brevets**

(11) **EP 4 756 410 A1**

(12) **EUROPEAN PATENT APPLICATION**
published in accordance with Art. 153(4) EPC

(43) Date of publication:
**10.06.2026 Bulletin 2026/24**

(21) Application number: **24849329.8**

(22) Date of filing: **19.04.2024**

(51) International Patent Classification (IPC):
*G01N 21/84* (2006.01)     *G01N 33/487* (2006.01)
*G06T 7/00* (2017.01)      *G06T 7/11* (2017.01)
*G01N 21/17* (2006.01)

(52) Cooperative Patent Classification (CPC):
**G01N 21/17; G01N 21/84; G01N 33/487;**
**G06T 7/00; G06T 7/11**

(86) International application number:
**PCT/KR2024/005338**

(87) International publication number:
**WO 2025/028757 (06.02.2025 Gazette 2025/06)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority:  **02.08.2023  KR 20230101245**

(71) Applicant: **INTIN INC.**
**Daegu 41069 (KR)**

(72) Inventors:
• **KIM, Ji Hoon**
**Seoul 06981 (KR)**
• **HONG, Chung Pyo**
**Seoul 03763 (KR)**

(74) Representative: **Metida**
**Gyneju str. 16**
**01109 Vilnius (LT)**

(54) **BODY FLUID TESTING METHOD AND APPARATUS THEREFOR**

(57)   The present disclosure relates to a body fluid testing method including: identifying a region of interest (ROI) for each sperm in each of a set n image frames; adjusting brightness of each of the n image frames to a lower level by a preset value; generating a single accumulated image frame by accumulating images of each sperm within the n image frames; identifying an area of a movement path for each sperm in the accumulated image frame; calculating an area of the movement path for each sperm and determining motility based on the calculated area; and calculating a width-to-height ratio of a rectangle enclosing the area of the movement path for each sperm and determining straightness based on the calculated width-to-height ratio.

**FIG. 5**

IDENTIFYING ROI IN SET N IMAGE FRAMES — S501

PERFORMING IMAGE PREPROCESSING FOR EACH OF N IMAGE FRAMES — S502

ADJUSTING BRIGHTNESS OF N IMAGE FRAMES TO LOWER LEVEL BY PRESET VALUE — S503

GENERATING SINGLE ACCUMULATED IMAGE FRAME BY ACCUMULATING SPERM IMAGES WITHIN N IMAGE FRAMES — S504

IDENTIFYING MOVEMENT PATH FOR EACH SPERM IN ACCUMULATED IMAGE FRAME — S505

IDENTIFYING DEGREE OF MOTILITY BASED ON AREA (SURFACE AREA) OF MOVEMENT PATH REGION OF EACH SPERM — S506

IDENTIFYING DEGREE OF STRAIGHTNESS BY DETERMINING WIDTH-TO-HEIGHT RATIO OF MOVEMENT PATH REGION OF EACH SPERM — S507

GENERATING AND OUTPUTTING TEST RESULT — S508

**Description**

[Technical Field]

The present disclosure relates to a body fluid testing method and apparatus therefor, and more particularly, to a body fluid testing method and apparatus therefor capable of rapidly analyzing a semen image photographed by a hospital testing apparatus, a portable testing apparatus, or a mobile application.

[Background Art]

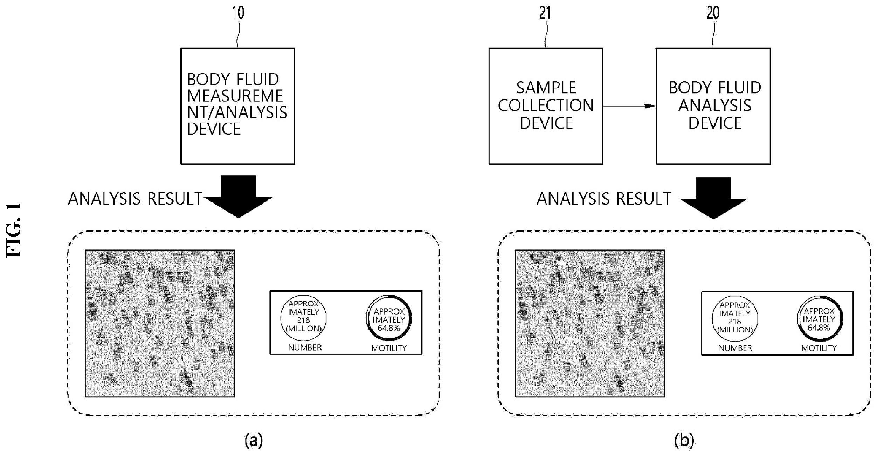

In general, a semen test is performed by a single body fluid measurement/analysis device 10 that collects semen, photographs the collected semen, examines the number of semen photographed and the movement of each semen, and outputs an analysis result (test result) on a screen, as shown in (a) of FIG. 1.

Alternatively, as illustrated in (b) of FIG. 1, the semen test is performed by collecting semen from a sample collection device 21, photographing the collected semen through the sample collection device 21 itself or an external photographing device, providing the photographed semen image (or video) to a body fluid analysis device 20, examining, at the body fluid analysis device 20, the number of semen and the movement of each semen, and outputting an analysis result on a screen.

At this time, the semen testing method includes photographing semen collected as a sample to create an image, identifying an ROI (Region of Interest) (i.e., a sperm region) in the semen image, and performing frame-by-frame tracker analysis of the semen image to analyze movement presence, movement degree, and movement direction of sperm.

However, since the conventional semen testing method analyzes movement presence, movement degree, and movement direction of each sperm in every set image frame, the test time required for ROI search, tracker analysis, and result processing and output takes about 88 seconds on average.

However, when the conventional semen testing method is applied to a mobile device, 88 seconds may be perceived as a long time for a mobile device user who desires to check examination results more quickly.

Accordingly, there is a need for a solution capable of performing a semen test in a shorter time.

[Disclosure]

[Technical Problem]

The present disclosure provides a body fluid testing method and apparatus therefor that enable semen testing to be performed in a shorter time than conventional methods.

[Technical Solution]

In order to achieve the above-described object, a body fluid testing method performed by a body fluid testing apparatus according to an embodiment of the present disclosure may include: identifying a region of interest (ROI) for each sperm in each of a set n image frames; adjusting brightness of each of the n image frames to a lower level by a preset value; generating a single accumulated image frame by accumulating images of each sperm within the n image frames; identifying an area of a movement path for each sperm in the accumulated image frame; calculating an area of the movement path for each sperm and determining motility based on the calculated area of each sperm; and calculating a width-to-height ratio of a rectangle enclosing the area of the movement path for each sperm and determining straightness based on the calculated width-to-height ratio.

The body fluid testing method according to an embodiment of the present disclosure may include performing a preprocessing operation to adjust brightness and contrast for each of the n image frames.

The preset value may be (1/n).

In determining the motility, sperm may not be recognized when the calculated area is smaller than a first reference area, stationary sperm may be determined when the calculated area is greater than the first reference area and smaller than a second reference area, and motile sperm may be determined when the calculated area is greater than or equal to the second reference area.

In determining the straightness, straight sperm may be determined when the calculated width-to-height ratio is greater than or equal to a preset ratio.

The body fluid testing method according to an embodiment of the present disclosure may further include calculating a motility value and a straightness value for sperm of a corresponding test target using the number of stationary sperm, the number of the motile sperm, and the number of straight sperm.

In order to achieve the above-described object, a body fluid testing apparatus according to an embodiment of the present disclosure may include a memory and a processor connected to the memory and configured to execute instructions stored in the memory.

The processor may be configured to: identify a region of interest (ROI) for each sperm in each of a set n image frames; adjust brightness of each of the n image frames to a lower level by a preset value; generate a single accumulated image frame by accumulating images of each sperm within the n image frames; identify an area of a movement path for each sperm in the accumulated image frame; calculate an area of the movement path for each sperm and determining motility based on the calculated area of each sperm; and calculate a width-to-height ratio of a rectangle enclosing the area of the movement path for each sperm and determine straightness based on the calculated width-to-height ratio.

The processor may be further configured to perform a preprocessing operation for noise removal for each of the n image frames.

The body fluid testing apparatus according to an embodiment of the present disclosure may further include a photographing unit that photographs a subject and stores the photographed image in the memory.

The body fluid testing apparatus according to an embodiment of the present disclosure may further include a chamber unit that stores a semen sample collected from an individual.

The photographing unit may be configured to photograph a semen sample stored in the chamber unit.

The processor may be further configured to calculate a motility value and a straightness value for sperm of a corresponding test target using the number of stationary sperm, the number of motile sperm, and the number of linear sperm.

[Advantageous Effects]

The present disclosure has the advantage of significantly reducing a semen testing time by accumulating movement for each frame of a semen image and analyzing the semen image using accumulated results.

[Description of Drawings]

FIG. 1 is a diagram illustrating a body fluid testing apparatus according to a conventional embodiment.

FIG. 2 is a block diagram of a body fluid testing apparatus according to a first embodiment of the present disclosure.

FIG. 3 is a diagram illustrating a process of accumulating images according to an embodiment of the present disclosure.

FIG. 4 is a block diagram of a body fluid testing apparatus according to a second embodiment of the present disclosure.

FIG. 5 is a schematic flowchart of a body fluid testing method according to an embodiment of the present disclosure.

FIG. 6 is a diagram illustrating determination of sperm motility according to an embodiment of the present disclosure.

FIG. 7 is a diagram illustrating determination of sperm straightness according to an embodiment of the present disclosure.

FIG. 8 is a diagram illustrating results of a clinical image verification test according to an embodiment of the present disclosure.

[Mode for Carrying Out the Invention]

Hereinafter, embodiments disclosed in this specification will be described in detail with reference to the accompanying drawings. Regardless of the reference numerals, identical or similar components are denoted by the same reference numerals, and redundant descriptions thereof will be omitted. In addition, when describing the embodiments disclosed in this specification, if it is determined that a detailed description of a related known technology may obscure the gist of the embodiments disclosed herein, a detailed description thereof will be omitted.

Terms including ordinal numbers, such as first, second, and the like, may be used to describe various components, but the components are not limited by the terms. The above terms are used solely to distinguish one component from another.

Singular expressions include plural expressions unless the context clearly indicates otherwise.

In this application, each step described may be performed regardless of the described order, except in cases where a specific causal relationship requires that the steps be performed in the described order.

In this application, terms such as "include" or "have" are intended to specify the presence of a feature, number, step, operation, component, part, or combination thereof described in the specification, but should be understood not to exclude in advance the possibility of the presence or addition of one or more other features, numbers, steps, operations, components, parts, or combinations thereof.

The embodiments of the present disclosure will now be described in detail with reference to the accompanying drawings.

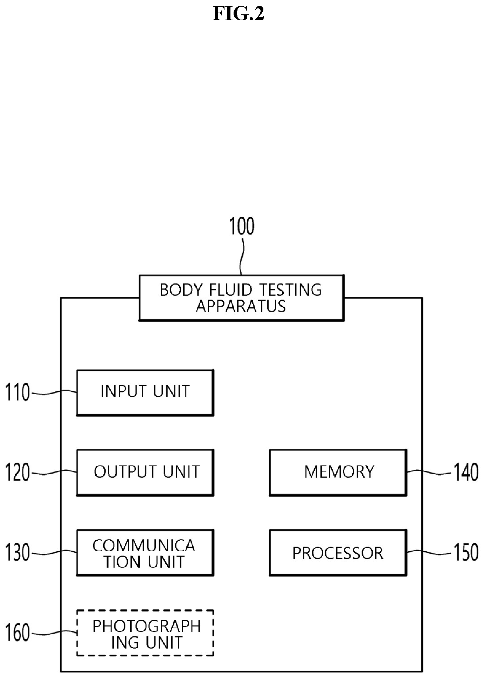

FIG. 2 is a block diagram of a body fluid testing apparatus according to a first embodiment of the present disclosure. Referring to FIG. 2, a body fluid testing apparatus 100 according to a first embodiment of the present disclosure may include an input unit 110, an output unit 120, a communication unit 130, a memory 140, and a processor 150. The body fluid testing apparatus 100 according to the first embodiment of the present disclosure may further include a photographing unit 160.

Such a body fluid testing apparatus 100 may be a device used in a hospital or a portable computer device such as a mobile phone or a laptop.

The input unit 110 may receive various information through a user's operations and input actions. The input unit 110 may be a touch screen module, a keyboard, a mouse, a button, a stylus, a microphone, or the like.

The output unit 120 may output various information. The output unit 120 may be a display device, a speaker, a vibration generating device, a tactile generating device, or the like.

The communication unit 130 enables the body fluid testing apparatus 100 to communicate with an external device via a network in a wired or wireless manner. The communication unit 130 may receive, from outside, a body fluid image that is subject to body fluid testing

The memory 140 functions as a storage medium and may store a plurality of application programs executed in the body fluid testing apparatus 100, semen images subject to body fluid testing, and data and commands for the operation of the body fluid testing apparatus 100. This memory may be implemented in hardware in the form of various storage devices such as ROM, RAM, flash drives, and hard drives, or in the form of web storage.

The photographing unit 160 is a camera device that enables photographing of a subject. The photographing unit 160 may perform the function of photographing a body fluid accommodated in an external body fluid collection device in conjunction with a body fluid testing program among the applications. A body fluid (semen) image photographed by the photographing unit 160 is stored in the memory 140.

The processor 150 controls the overall operations of the input unit 110, the output unit 120, the communication unit 130, and the memory 140, and in some cases, the photographing unit 160, thereby enabling body fluid testing. The processor 150 may examine a semen image received through the communication unit 130 or examine a semen image photographed by the photographing unit 160.

The processor 150 sequentially performs an ROI identification process, an image brightness adjustment process, a frame accumulation process, an image analysis process, and a result output process for a semen image. In addition, the processor 150 may further perform an image preprocessing process to facilitate image analysis before the ROI identification process. The image preprocessing process is a process for adjusting brightness and contrast. Of course, the image preprocessing process may further include a noise removal process.

The result output process may be performed by a separate apparatus.

The ROI identification process is a process that has been commonly used in conventional semen testing methods, and is a process of searching for and identifying an area (i.e., region of interest) where each sperm is located in each frame of a semen image. The image brightness adjustment process adjusts the image brightness by a preset value for each image frame required for semen testing. In adjusting the brightness of the image to a lower level, when each of the set number of frames is accumulated, the brightness of the image increases, and as a result, the brightness becomes excessively high, making it impossible for the processor 150 to identify an object. Therefore, the processor 150 adjusts the image brightness of each frame by a preset value so that the brightness of the accumulated frames corresponds to the brightness of the received frame or falls within a preset range. The preset value may be defined as the brightness of the current frame multiplied by (1 / the number of image frames set).

The frame accumulation process refers to a process of accumulating a set number of image frames in chronological order to form a single frame. For example, in a first method of accumulating a set number of image frames to form one frame, when three frames are used, an object of a first frame (i.e., an image of sperm) is reflected (or accumulated) into a second frame, and an object of the second frame, in which the object of the first frame has been accumulated, is further accumulated into a third frame. Alternatively, in a second method, in contrast to the first method, an object of a second frame is accumulated into a first frame, and an object of a third frame is also accumulated into the first frame. Therefore, in the first method, the last frame becomes one image in which the objects of the respective frames are accumulated, and in the second method, the first frame becomes one image in which the objects of the respective frames are accumulated.

Alternatively, in a third method, objects of the first, second, and third frames are reflected into a newly generated frame so that the objects of the respective frames are accumulated into the new frame.

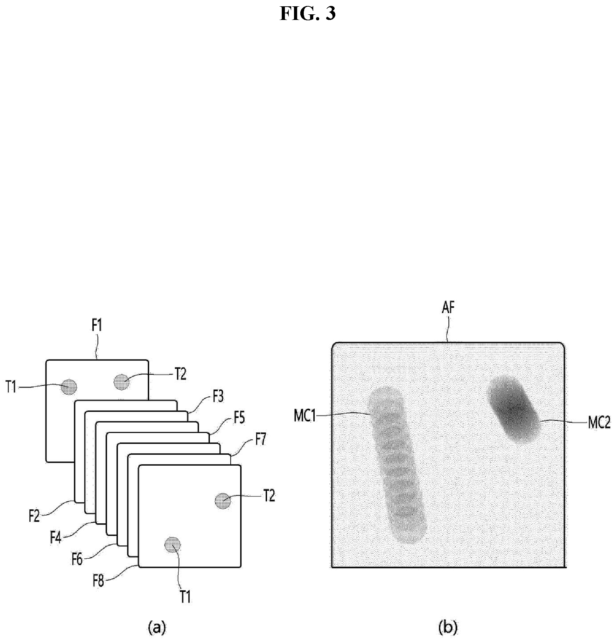

Accumulating objects in a frame will be described with reference to FIG. 3. FIG. 3 is a diagram illustrating a process of accumulating images according to an embodiment of the present disclosure.

It is assumed that there are eight image frames F1 to F8 for semen testing in FIG. 3(a), and that images of a first sperm T1 and a second sperm T2 are displayed in each frame. Since sperm are motile, the first sperm T1 and the second sperm T2 in a first frame F1 move as time passes, and accordingly move to positions in an eighth frame F8, which are different from those in the first frame F1.

When images of the first sperm T1 and the second sperm T2 displayed in the eight image frames F1 to F8 are accumulated and displayed in a single frame, an accumulated image MC1 for the first sperm T1 and an accumulated image MC2 for the second sperm T2 may be obtained, as shown in (b) of FIG. 3. It can be seen that the accumulated images MC1 and MC2 represent the movement trajectories of sperm, that is, the movement paths thereof. Hereinafter, an accumulated image of sperm are referred to as a "movement path" thereof.

An image analysis process refers to a process of analyzing motility and straightness of each object (i.e., sperm) by using the movement path of sperm to identify the number of stationary sperm, motile sperm, and straight sperm. In addition, the image analysis process may further include a process of calculating a motility value (a degree of motility) and a straightness value (a degree of straightness) for sperm of a corresponding test target by using the number of stationary sperm, motile sperm, and straight sperm.

Here, a stationary sperm refers to a sperm having neither motility nor straightness, which means that a recognized object is large enough to be recognized as a sperm but exhibits little movement. An motile sperm refers to a sperm that has motility, which means that the recognized object is large enough to be recognized as a sperm and has a large movement path but does not move in a straight line. A straight sperm refers to a sperm that has straightness, which means that a recognized object is large enough to be recognized as a sperm, has a large movement path, and has a long trajectory extending horizontally or vertically.

A motility value is an indicator representing the degree of motility exhibited by sperm, and a straightness value is an indicator representing the degree of straightness in sperm movement. The motility value represents a value obtained by dividing a first number, which is the sum of the numbers of motile and straight sperm, by a second number, which is the sum of the numbers of motile, straight, and stationary sperm, as shown in Equation 1.

(Equation 1)

$$Motility\ value$$
$$= \frac{Number\ of\ motile\ sperm + Number\ of\ straight\ sperm}{Number\ of\ motile\ sperm + Number\ of\ straight\ sperm + Number\ of\ stationary\ sperm}$$

In addition, the straightness value represents a value obtained by dividing the number of straight sperm by the second number, as shown in Equation 2.

(Equation 2)

$$Straightness\ value$$
$$= \frac{Number\ of\ straight\ sperm}{Number\ of\ motile\ sperm + Number\ of\ straight\ sperm + Number\ of\ stationary\ sperm}$$

Meanwhile, in the image analysis process, an activation rate may be calculated. The activation rate is an indicator representing how much faster or slower the average sperm velocity of a test target is compared with the average sperm velocity of the general population. The activation rate ($P_{activation}$) may be calculated using Equation 3 below.

(Equation 3)

$$P_{activation} = \frac{\frac{\sum Area(irregular,\ straight)}{\sum Num(irregular,\ straight)} \times \frac{FPS\ of\ video}{F_{number\ of\ measurement\ frames}}}{V_{avg} \times C_0}$$

In Equation 3, $\Sigma Area$ (*irregular, straight*) represents the total movement area (width) of irregularly moving objects (i.e., sperm) and straight-moving objects (i.e., sperm), and the unit is $PIXEL^2$. In Equation 3, $\Sigma Num$ (*irregular, straight*) represents the total number of irregularly moving objects and straight-moving objects, and it has no unit. $V_{avg}$ denotes the average movement velocity of sperm, and the unit is $\mu m/sec$. $C_0$ is a coefficient for converting area into length, and its unit is $PIXEL^2/\mu m$. The unit thereof is $PIXEL2/\mu m$.

If the activation rate ($P_{activation}$) is 2, this indicates that the average sperm velocity of the test target is twice the average sperm velocity of the general population.

Meanwhile, since Co represents a very small length, it can be approximated as $PIXEL/\mu m$ without significant error, and thus may be calculated by approximation.

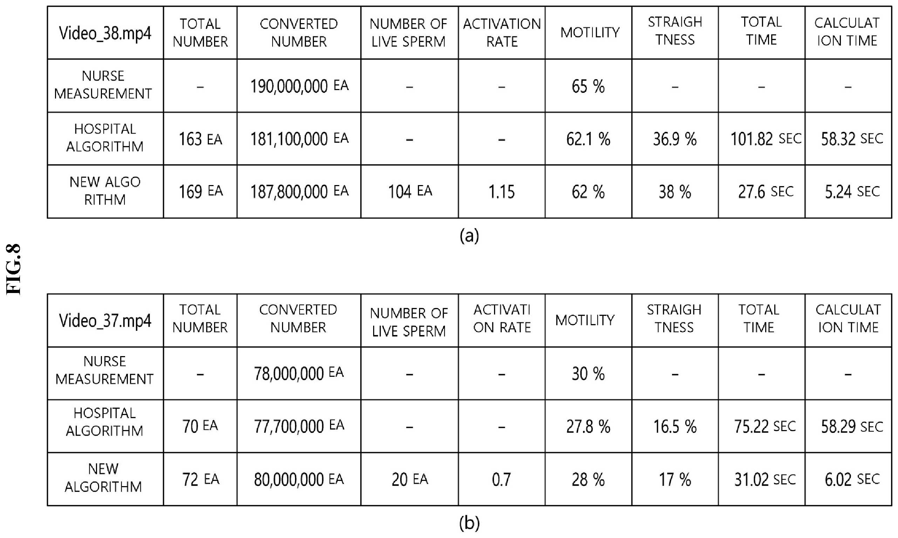

A result output process may output analysis results obtained in the image analysis process through an output unit 120. The analysis results may be presented in the form of an image or a table (see FIG. 8).

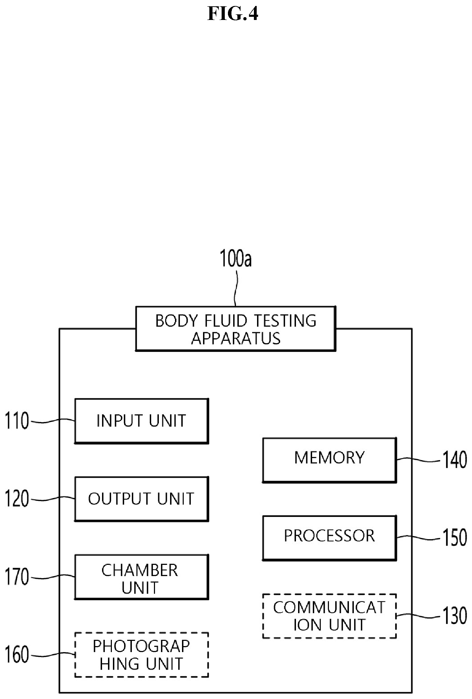

FIG. 4 is a block diagram of a body fluid testing apparatus according to a second embodiment of the present disclosure. Referring to FIG. 4, a body fluid testing apparatus 100a according to the second embodiment of the present disclosure may include an input unit 110, an output unit 120, a chamber unit 170, a memory 140, and a processor 150. In addition, the body fluid testing apparatus 100a according to the second embodiment may further include at least one of a photographing unit 160 and a communication unit 130.

The input unit 110, the output unit 120, the communication unit 130, the memory 140, and the processor 150 of the body fluid testing apparatus 100a according to the second embodiment are identical to the corresponding components of the body fluid testing apparatus 100 according to the first embodiment of the present disclosure.

The chamber unit 170 is configured to store a semen sample collected from an individual and to allow the sample to be photographed by the photographing unit 160. The photographing unit 150 according to the second embodiment is configured to photograph an image of semen stored in the chamber unit 170, and the photographed image is stored in the memory 140.

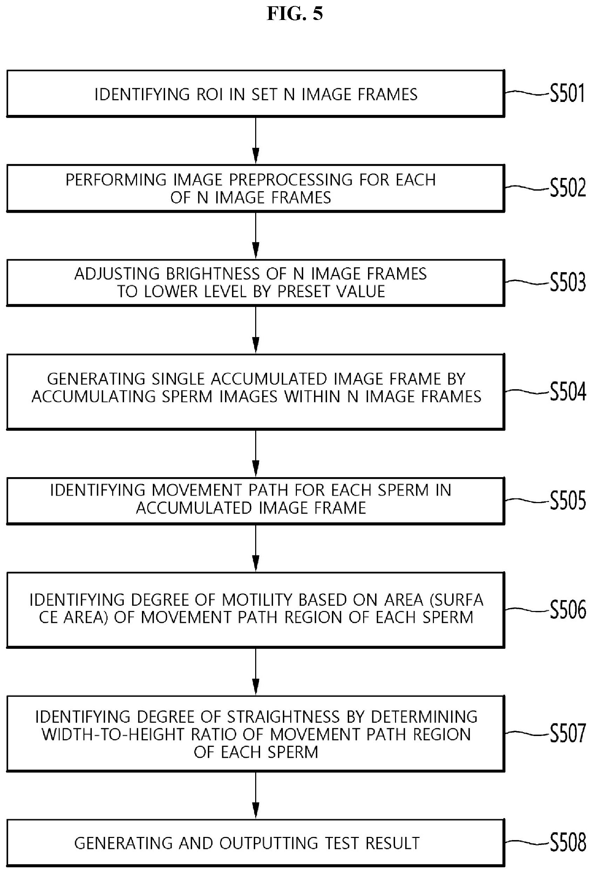

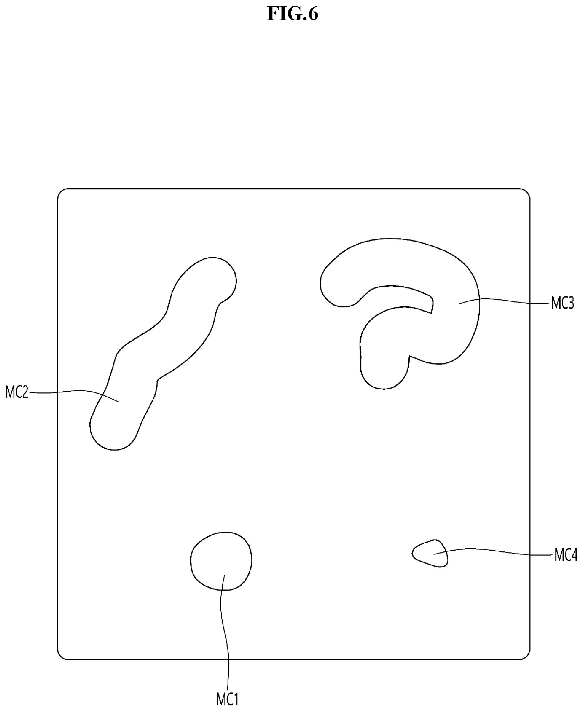

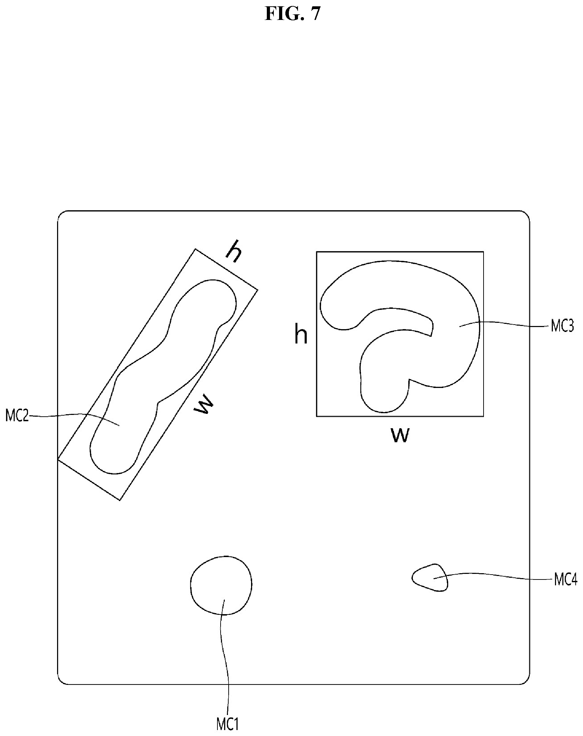

Hereinafter, a body fluid testing method according to an embodiment of the present disclosure will be described with reference to FIGS. 5 to 8. FIG. 5 is a schematic flowchart of a body fluid testing method according to an embodiment of the present disclosure, FIG. 6 is a diagram illustrating a determination of sperm motility according to an embodiment of the present disclosure, and FIG. 7 is a diagram illustrating a determination of sperm straightness according to an embodiment of the present disclosure.

In FIG. 5, n image frames may be image frames provided from an external source or image frames photographed internally, and are temporally continuous image frames.

In operation S501, a processor 150 performs a pre-processing operation for adjusting brightness and contrast for each of the n image frames that are subject to body fluid testing.

In operation S502, the processor 150 searches for and identifies an ROI region for each sperm in each of the n image frames set for semen examination. Through this ROI identification, each sperm is identified on a frame-by-frame basis.

In operation S503, the processor 150 reduces the brightness of each of the n image frames by a preset value. For example, if the preset value is 1/10, the processor 150 reduces the brightness of each image frame by one-tenth. As another example, the processor 150 may calculate the preset value based on the number of image frames. For instance, if the number of image frames subject to body fluid testing is 30, the processor 150 may set the preset value to 1/30.

In operation S504, the processor 150 generates a single accumulated image frame by accumulating the images of each sperm within the n image frames. For example, when there are eight image frames, the ROI of each sperm in the first image frame is represented in a first accumulated image frame, the ROI of each sperm in the second image frame (i.e., the image of each sperm) is also represented in the first accumulated image frame, and the ROIs of each sperm in the third to eighth subsequent frames are sequentially represented in the first accumulated image frame. Accordingly, the ROI of each sperm is accumulated and displayed in chronological order, as illustrated in FIG. 4(b), and thus appears as a movement trajectory of each sperm.

In operation S505, the processor 150 identifies a movement path for each sperm in the accumulated image frame.

In operation S506, the processor 150 identifies the area of the movement path for each sperm, calculates the size of the area, and compares the calculated area with a first reference area and a second reference area to determine the motility of each sperm. If the area of a movement path of an object is smaller than the first reference area, the processor 150 does not determine the object to be sperm, and if the area is greater than the first reference area and smaller than the second reference area, the processor 150 determines the object to be sperm but classifies the object as stationary sperm because the object is immotile. In addition, if the area of the movement path is equal to or greater than the second reference area, the processor 150 determines the object to be a motile sperm. Accordingly, in operation S506, the numbers of stationary and motile sperm of a test target are obtained.<

In operation S507, the processor 150 determines an area of a movement path of each sperm, forms a rectangle that encloses the area, calculates a ratio of a width and a height of the rectangle (a width/height ratio), and subsequently compares the calculated ratio with a preset ratio to determine straightness. The rectangle may be a minimum rectangle that encloses the movement path. When the width-to-height ratio of the movement path is equal to or greater than the preset ratio, the processor 150 determines the corresponding sperm to be straight sperm. Accordingly, in operation S507, the number of straight sperm of the test target is obtained.

In addition, through operations S506 and S507, the processor 150 identifies the numbers of motile and straight sperm of a test target and also identifies the number of stationary sperm by determining sperm that lack both motility and straightness to be stationary sperm.

In operation S508, the processor 150 uses the numbers of stationary, motile, and straight sperm to calculate at least one of a motility value, a straightness value, or an activation rate as a test result for a test target, and outputs the result through an output unit 120. Here, the test result may include the number of sperm identified through ROI recognition, sperm motility, sperm straightness, and the number of live sperm identified based on sperm motility and straightness.

In FIG. 6, a first movement path MC1 is determined to be stationary sperm because the area thereof is larger than the first reference area but smaller than the second reference area. In addition, a second movement path MC2 and a third movement path MC3 are determined to be motile sperm because the areas thereof are sufficiently large, that is, greater than the second reference area. A fourth movement path MC4 is not determined to be sperm because the area thereof is too small, that is, less than or equal to the first reference area.

In FIG. 7, the first movement path MC1 corresponds to stationary sperm, and the fourth movement path MC4 is not sperm, and therefore, both are excluded from the target for determining straightness.

In addition, the second movement path MC2 is determined to be straight sperm having straightness, because the ratio of the width w to the height h of a minimum rectangle enclosing the movement path MC2 is approximately 5:1 or greater. However, the third movement path MC3 is determined not to have straightness, because the ratio of the width w to the height h of a minimum rectangle enclosing the movement path MC3 is approximately 1:1.

FIG. 8 is a diagram illustrating results of a clinical image verification test according to an embodiment of the present disclosure. In (a) and (b) of FIG. 8 are illustrated the results of visual measurement by a nurse, the results obtained using a hospital semen-testing algorithm, and the results obtained using a body fluid testing method and apparatus according to an embodiment of the present disclosure. The total number refers to the number of sperm within a designated area, and the converted count refers to the number of sperm obtained by converting the total number to correspond to the entire area. The total time refers to the overall time required to perform a semen analysis, and the calculation time refers to the tracker-calculation time.

As seen in (a) and (b) of FIG. 8, the motility and straightness obtained using the body fluid testing method and apparatus are substantially similar to the motility and straightness values obtained using the hospital algorithm.

However, it can be seen that the tracker-calculation time is significantly shorter at 5.24 seconds or 6.02 seconds when applying the method of the present disclosure, as compared to 58.32 seconds or 58.29 seconds when using the hospital algorithm. Accordingly, the total calculation time is also significantly shortened, from 101.82 seconds or 75.22 seconds when using the hospital algorithm to 27.6 seconds or 31.02 seconds when applying the method of the present disclosure.

The technical features disclosed in the respective embodiments of the present disclosure are not limited to those embodiments, and, unless mutually incompatible, the technical features disclosed in the embodiments may be variously combined and implemented in different embodiments.

Therefore, although each embodiment is described focusing on its respective technical features, the technical features may be applied in combination with one another as long as they are not mutually incompatible.

The present disclosure is not limited to the above-described embodiments and the accompanying drawings, and various modifications and variations can be made by those skilled in the art. Therefore, the scope of the present disclosure should be defined not only by the claims set forth in this specification but also by equivalents thereof.

**Claims**

1.  A body fluid testing method performed by a body fluid testing apparatus, the method comprising:

    identifying a region of interest (ROI) for each sperm in each of a set n image frames;
    adjusting brightness of each of the n image frames to a lower level by a preset value;
    generating a single accumulated image frame by accumulating images of each sperm within the n image frames;
    identifying an area of a movement path for each sperm in the accumulated image frame;
    calculating an area of the movement path for each sperm and determining motility based on the calculated area of each sperm; and
    calculating a width-to-height ratio of a rectangle enclosing the area of the movement path for each sperm and determining straightness based on the calculated width-to-height ratio.

2. The body fluid testing method of claim 1, further comprising performing a preprocessing operation to adjust brightness and contrast for each of the n image frames.

3. The body fluid testing method of claim 1, wherein the preset value is (1/n).

4. The body fluid testing method of claim 1, wherein, in determining the motility, sperm is not recognized when the calculated area is smaller than a first reference area, stationary sperm is determined when the calculated area is greater than the first reference area and smaller than a second reference area, and motile sperm is determined when the calculated area is greater than or equal to the second reference area.

5. The body fluid testing method of claim 4, wherein, in determining the straightness, straight sperm is determined when the calculated width-to-height ratio is greater than or equal to a preset ratio.

6. The body fluid testing method of claim 5, further comprising calculating a motility value and a straightness value for sperm of a corresponding test target using the number of stationary sperm, the number of the motile sperm, and the number of straight sperm.

7. A body fluid testing apparatus comprising:

a memory, and
a processor connected to the memory and configured to execute instructions stored in the memory,
wherein the processor is configured to:

identify a region of interest (ROI) for each sperm in each of a set n image frames;
adjust brightness of each of the n image frames to a lower level by a preset value;
generate a single accumulated image frame by accumulating images of each sperm within the n image frames;
identify an area of a movement path for each sperm in the accumulated image frame;
calculate an area of the movement path for each sperm and determining motility based on the calculated area of each sperm; and
calculate a width-to-height ratio of a rectangle enclosing the area of the movement path for each sperm and determine straightness based on the calculated width-to-height ratio.

8. The body fluid testing apparatus of claim 7, wherein the processor is further configured to perform a preprocessing operation for noise removal for each of the n image frames.

9. The body fluid testing apparatus of claim 7 or 8, further comprising a photographing unit that photographs a subject and stores the photographed image in the memory.

10. The body fluid testing apparatus of claim 9, further comprising a chamber unit that stores a semen sample collected from an individual.

11. The body fluid testing apparatus of claim 10, wherein the photographing unit is configured to photograph a semen sample stored in the chamber unit.

12. The body fluid testing apparatus of claim 7, wherein the processor is further configured to calculate a motility value and a straightness value for sperm of a corresponding test target using a number of stationary sperm, a number of motile sperm, and a number of straight sperm.

13. The body fluid testing apparatus of claim 7, wherein the preset value is (1/n).

# FIG. 1

**FIG.2**

100

BODY FLUID TESTING
APPARATUS

110 — INPUT UNIT

120 — OUTPUT UNIT

130 — COMMUNICA
TION UNIT

160 — PHOTOGRAPH
ING UNIT

MEMORY — 140

PROCESSOR — 150

# FIG. 3

(a)

(b)

**FIG.4**

**FIG. 5**

IDENTIFYING ROI IN SET N IMAGE FRAMES — S501

PERFORMING IMAGE PREPROCESSING FOR EACH
OF N IMAGE FRAMES — S502

ADJUSTING BRIGHTNESS OF N IMAGE FRAMES
TO LOWER LEVEL BY PRESET VALUE — S503

GENERATING SINGLE ACCUMULATED IMAGE FRAME BY
ACCUMULATING SPERM IMAGES WITHIN N IMAGE FRAMES — S504

IDENTIFYING MOVEMENT PATH FOR EACH SPERM IN
ACCUMULATED IMAGE FRAME — S505

IDENTIFYING DEGREE OF MOTILITY BASED ON AREA (SURFA
CE AREA) OF MOVEMENT PATH REGION OF EACH SPERM — S506

IDENTIFYING DEGREE OF STRAIGHTNESS BY DETERMINING
WIDTH-TO-HEIGHT RATIO OF MOVEMENT PATH REGION
OF EACH SPERM — S507

GENERATING AND OUTPUTTING TEST RESULT — S508

EP 4 756 410 A1

**FIG.6**

14

**FIG. 7**

## FIG.8

(a)

| Video_38.mp4 | TOTAL NUMBER | CONVERTED NUMBER | NUMBER OF LIVE SPERM | ACTIVATION RATE | MOTILITY | STRAIGHTNESS | TOTAL TIME | CALCULATION TIME |
|---|---|---|---|---|---|---|---|---|
| NURSE MEASUREMENT | – | 190,000,000 EA | – | – | 65 % | – | – | – |
| HOSPITAL ALGORITHM | 163 EA | 181,100,000 EA | – | – | 62.1 % | 36.9 % | 101.82 SEC | 58.32 SEC |
| NEW ALGORITHM | 169 EA | 187,800,000 EA | 104 EA | 1.15 | 62 % | 38 % | 27.6 SEC | 5.24 SEC |

(b)

| Video_37.mp4 | TOTAL NUMBER | CONVERTED NUMBER | NUMBER OF LIVE SPERM | ACTIVATION RATE | MOTILITY | STRAIGHTNESS | TOTAL TIME | CALCULATION TIME |
|---|---|---|---|---|---|---|---|---|
| NURSE MEASUREMENT | – | 78,000,000 EA | – | – | 30 % | – | – | – |
| HOSPITAL ALGORITHM | 70 EA | 77,700,000 EA | – | – | 27.8 % | 16.5 % | 75.22 SEC | 58.29 SEC |
| NEW ALGORITHM | 72 EA | 80,000,000 EA | 20 EA | 0.7 | 28 % | 17 % | 31.02 SEC | 6.02 SEC |

## INTERNATIONAL SEARCH REPORT

| International application No. |
| --- |
| **PCT/KR2024/005338** |

**A. CLASSIFICATION OF SUBJECT MATTER**

**G01N 21/84**(2006.01)i; **G01N 33/487**(2006.01)i; **G06T 7/00**(2006.01)i; **G06T 7/11**(2017.01)i; **G01N 21/17**(2006.01)i

According to International Patent Classification (IPC) or to both national classification and IPC

**B. FIELDS SEARCHED**

Minimum documentation searched (classification system followed by classification symbols)

G01N 21/84(2006.01); G01N 21/00(2006.01); G06K 9/00(2006.01); G06T 7/246(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 정자(sperm), 운동성(motility), 영역(area), 직진성(straightness), 및 경로(path)

**C. DOCUMENTS CONSIDERED TO BE RELEVANT**

| Category* | Citation of document, with indication, where appropriate, of the relevant passages | Relevant to claim No. |
| --- | --- | --- |
| Y | WILSON-LEEDY, Jonas G. et al. Development of a novel CASA system based on open source software for characterization of zebrafish sperm motility parameters. Theriogenology. 2007, vol. 67, pp. 661-672. See pages 662-667 and figures 1-5. | 1-13 |
| Y | TW 201229510 A (CENTRAL TAIWAN UNIVERSITY OF SCIENCE AND TECHNOLOGY) 16 July 2012. See inner pages 4-5 and figures 1-7 and 10-12. | 1-13 |
| Y | CN 104237121 A (SHANDONG UNIVERSITY) 24 December 2014 (2014-12-24) See paragraphs [0047]-[0049] and [0074]-[0075] and figures 1-3. | 2,8 |
| A | MAZZILLI, Fernando et al. Superimposed Image Analysis System (SIAS)* software: a new approach to sperm motility assessment. Techniques and instrumentation. September 1995, vol. 64, no. 3, pp. 653-656. See pages 653-656 and figures 1-3. | 1-13 |
| A | CN 112580476 A (SHENZHEN CHUANGHUAI MEDICAL TECHNOLOGY CO., LTD.) 30 March 2021 (2021-03-30) See paragraphs [0041]-[0157] and figures 1-21. | 1-13 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **23 July 2024** | **23 July 2024** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/005338**

| Patent document cited in search report | | | Publication date (day/month/year) | Patent family member(s) | | | Publication date (day/month/year) |
|---|---|---|---|---|---|---|---|
| TW | 201229510 | A | | None | | | |
| CN | 104237121 | A | 24 December 2014 | CN | 104237121 | B | 01 June 2016 |
| CN | 112580476 | A | 30 March 2021 | CN | 112580476 | B | 22 March 2024 |

Form PCT/ISA/210 (patent family annex) (July 2022)